# EUROPEAN PATENT SPECIFICATION

(11) **EP 1 595 569 B1**
(45) Date of publication and mention of the grant of the patent: **06.02.2008**
(21) Application number: 05005479.0
(22) Date of filing: 14.03.2005
(51) Int. Cl.: A61M 25/10, A61M 25/00, A61M 29/02

(54) **Catheter with expandable body**
Katheter mit einem expandierbaren Körper
Cathéter doté d'une partie extensible

(30) Priority: 26.03.2004 JP 2004093145
(43) Date of publication of application: 16.11.2005
(73) Proprietor: TERUMO KABUSHIKI KAISHA, Tokyo (JP)
(72) Inventor: Kumoyama, Kenichi, Fujinomiya-shi, Shizuoka 418-0015 (JP); Itou, Takenari, Fujinomiya-shi, Shizuoka 418-0015 (JP)
(74) Representative: Casalonga, Axel

(56) References cited:
- WO-A-01/45781
- WO-A-01/49337
- WO-A-96/14895
- US-A- 5 693 014
- US-A1- 2001 035 456

## Description

The present invention relates to a catheter with an expandable body for use in a treatment including an operation of dilating a constricted portion generated in a body lumen.

In a catheter with an expandable body for dilating a constricted portion (stenosed portion) generated in a blood vessel to thereby improve the bloodstream in the cases of PTCA (percutaneous transluminal coronary angioplasty), PTA (percutaneous transluminal angioplasty) and the like, an expandable body (balloon) is preliminarily folded onto a catheter shaft for permitting insertion into the blood vessel.

In recent years, there have been an increasing number of cases in which, after dilating a constricted portion by a catheter with an expandable body and evulsing the catheter with the expandable body from the constricted portion, the dilated state of the constricted portion is observed by use of an ultrasonic catheter or the like, and, when constriction remains, the catheter with the expandable body once evulsed is again inserted into the constricted portion to perform re-dilation.

However, when the expandable body is once expanded at a high pressure so as to dilate the constricted portion, the expandable body would not return to the original folded shape and would easily assume a flat war fan-like (wing-like) shape, after the expanding fluid is discharged from the expandable body to contract the expandable body. When the expandable body assumes such a war fan-like shape, it is difficult to reinsert the once evulsed catheter with the expandable body into the blood vessel.

In addition, with a conventional catheter with an expandable body, there have been cases in which the expandable body would slip off from the constricted portion at the time of expanding the expandable body for dilating the constricted portion. In such a case, the operations of once contracting the expandable body, then positioning the balloon to an appropriate position (the position of the constricted portion) and thereafter expanding the expandable body again, and, further, an operation of replacing the catheter with the expandable body by another catheter with an expandable body differing from the former in expandable body size, must be carried out; the operations take much labor and time, thereby impeding a smooth treatment and increasing the burden on the patient. It may be contemplated to lower the expanding pressure for the expandable body for the purpose of preventing the expandable body from coming off from the constricted portion, In this case, however, it is impossible to sufficiently dilate the constricted portion, and it may be impossible to expect the desired treatment effect.

It is known by WO 01/45781, a catheter balloon comprising a balloon body having a degree of expansion-contraction property, and areas spaced from each others in circumferential direction. Some areas have a high friction coefficient, the others having a low friction coefficient in order to prevent a slipping of the catheter and to make easier the insertion into the lumen body, respectively.

WO 96/14895 discloses a catheter balloon comprising an inflatable balloon mounted at the distal end of an elongated flexible shaft, and a flexible coating provided on the outer surface of the balloon which causes said balloon to prefer a predetermined trifold configuration, when a working fluid is sucked out of the expandable balloon.

It is also known by US 2001/0035456, a drug delivery device comprising an expandable catheter having an expandable portion, an expandable sheath which is disposed around the expandable portion, said sheath having a plurality of longitudinal slits or perforations. When the expandable portion is in an expanded state, the sheath is also in an expanded state and the perforations become open. The opening of the perforations enables to deliver a drug agent which is holded by the expandable portion of catheter.

Japanese Patent Laid-open No. Hei 5-337189 (EP553960A1) discloses a balloon catheter in which a tubular elastic membrane disposed around a balloon (expandable body) is provided between the balloon and a stent, and, upon contraction of the balloon, the elastic membrane squashes the balloon evenly in the circumferential direction, thereby preventing the balloon from assuming a flat war fan-like shape (refer, particularly, to paragraphs 0008 and 0022). However, since the elastic membrane is a simple tube, it is limited in expandability in the radial direction, and, when its outside diameter upon contraction of the balloon is set small for ensuring easy insertion into a blood vessel, it may obstruct the expansion of the balloon. Besides, Japanese Patent Laid-open No. Hei 5-337189 (EP553960A1) does not describe any consideration about the prevention of slipping-off of the expandable body from the constricted portion, in connection with the balloon catheter.

The present invention provides a catheter with an expandable body which is excellent in the property for re-insertion into a constricted portion in a body lumen. The present invention also provides a catheter with an expandable body which can assuredly prevent the expandable body from slipping off from a constricted portion at the time of dilating the constricted portion. Furthermore, the present invention provides a catheter with an expandable body which is excellent in both the property for re-insertion into a constricted portion and the prevention of slipping-off of the expandable body from the constricted portion.

According to the present invention, there is provided: a catheter including a shaft having a passage for permitting a working fluid to pass therethrough, an expandable body which is capable of expansion and contraction and provided at a distal end portion of the shaft so as to be communicated with the inside of the passage. The catheter includes an elastic tubular outer sleeve provided with at least one slit and fastened to the outside of the expandable body, the tubular outer sleeve having such a degree of extension-contraction property as to permit expansion of the expandable body when the working fluid is made to flow through the passage into the expendable body and to permit elastic contraction when said working fluid is sucked out of the expendable body to contract the expendable body into a compact form.

In the present invention, the outer sleeve may be in close contact with the outside surface of the expandable body.

The expandable body may be provided in the state of being folded on the distal end portion of the shaft before expanded.

Preferably, the inside diameter of the outer sleeve in the natural state is not more than the maximum outside diameter of the expanded body in the folded state. A distal end portion of the outer sleeve may be attached to a distal end portion of the expandable body and/or the shaft. A proximal end portion of the outer sleeve may be attached to a proximal end portion of the expandable body and/or the shaft.

The slit may be formed in parallel to the longitudinal direction of the outer sleeve or at an inclination relative to the longitudinal direction.

The outer sleeve may be provided with a plurality of the slits arranged intermittently along the circumferential direction of the outer sleeve. The outer sleeve may be provided with a plurality of the slits arranged intermittently along the longitudinal direction thereof. Preferably, the slit is formed in a spiral form, with the center axis of the outer sleeve as a center.

One of the outside surface of the outer sleeve and the outside surface of the expandable body has been subjected to a high lubricity treatment for displaying a comparatively high lubricity, and the other has been subjected to a low lubricity treatment for displaying a comparatively lower lubricity or has not been subjected to any lubricity treatment. Preferably, the outside surface of the outer sleeve has been subjected to the high lubricity treatment for displaying a comparatively high lubricity, and the outside surface of the expandable body has been subjected to the low lubricity treatment for displaying a comparatively lower lubricity. Also, preferably, the outside surface of the outer sleeve has been subjected to a lubricity treatment for displaying a lubricity, and the outside surface of the expandable body has not been subjected to any lubricity treatment.

A difference in the manner of formation of the slit(s) may exist between a distal end side portion and a proximal end side portion of the outer sleeve. Preferably, the difference in the manner of formation of the slit(s) is a difference or differences in at least one of the length, the width, the direction, the shape, and the formation density, of the slit(s).

According to the present invention, since the catheter with an expandable body includes the outer sleeve, upon contraction of the expandable body by sucking the working fluid out of the once expanded expandable body, a compressive force is exerted on the expandable body due to the elastic force of the outer sleeve, so that the expandable body can be contracted into a compact form.

Therefore, upon contraction of the expandable body from the expanded state, it is possible to securely prevent the expandable body from being squashed into a flat war fan-like (wing-like) shape; accordingly, when the catheter with the expandable body once evulsed from a constricted portion of a body lumen is again inserted into the constricted portion (stenosed portion), the re-insertion can be achieved smoothly and easily.

In addition, since the outer sleeve is provided with the slit(s) opened at the time of expansion of the expandable body, the outer sleeve can be easily bulged in the radial direction, so that the expandable body can be expanded sufficiently, without being obstructed by the outer sleeve.

Furthermore, when the expandable body is expanded to dilate a constricted portion (stenosed portion) of a body lumen, the contact surface, for contact with the constricted portion, formed by the outside surface of the expandable body exposed via the opened slit(s) and the outer sleeve is rugged (is including projections and recesses); so that friction between the contact surface and the constricted portion is enhanced, to display an anti-slipping effect. Therefore, even where the expandable body is expanded with a high pressure, the expandable body can be securely prevented from slipping off from the constricted portion.

The above and other features and advantages of the present invention will become apparent from the following description and appended claims, taken in conjunction with the accompanying drawings, in which:
Fig. 1 is a side view showing the surroundings of an expandable body in its contracted state in a first embodiment of the catheter with the expandable body according to the present invention;
Fig. 2 is a side view showing the surroundings of the expandable body in its expanded state in the catheter with the expandable body shown in Fig. 1;
Fig. 3 is a vertical sectional view showing the surroundings of the expandable body in its expanded state in the catheter with the expandable body shown in Fig. 1;
Fig. 4 is a cross-sectional view showing the expandable body in its contracted state and an inner tube, in the catheter with the expandable body shown in Fig. 1;
Fig. 5 is a cross-sectional view of the surroundings of the expandable body in its expanded state in the catheter with the expandable body shown in Fig. 1;
Fig. 6 is a side view for illustrating another configuration example of a slit(s) in an outer sleeve;
Fig. 7 is a side view showing the surroundings of an expandable body in its contracted state in a second embodiment of the catheter with the expandable body according to the present invention;
Fig. 8 is a side view showing the surroundings of an expandable body in its contracted state in a third embodiment of the catheter with the expandable body according to the present invention;
Fig. 9 is a side view showing the surroundings of an expandable body in its contracted state in a fourth embodiment of the catheter with the expandable body according to the present invention;
Fig. 10 is a vertical sectional view showing the surroundings of an expandable body in its expanded state in a fifth embodiment of the catheter with the expandable body according to the present invention;
Fig. 11 is a vertical sectional view showing the surroundings of an expandable body in its expanded state in a sixth embodiment of the catheter with the expandable body according to the present invention; and
Fig. 12 is a vertical sectional view showing the surroundings of an expandable body in its expanded state in a seventh embodiment of the catheter with the expandable body according to the present invention.

Now, the catheter with an expandable body according to the present invention will be described in detail below, based on some preferred embodiments shown in the accompanying drawings.

### <First Embodiment>

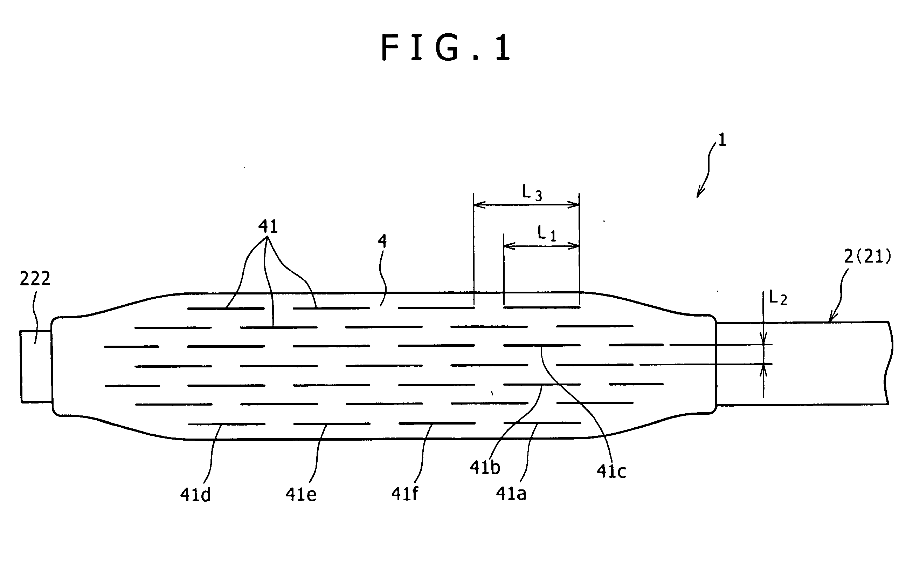

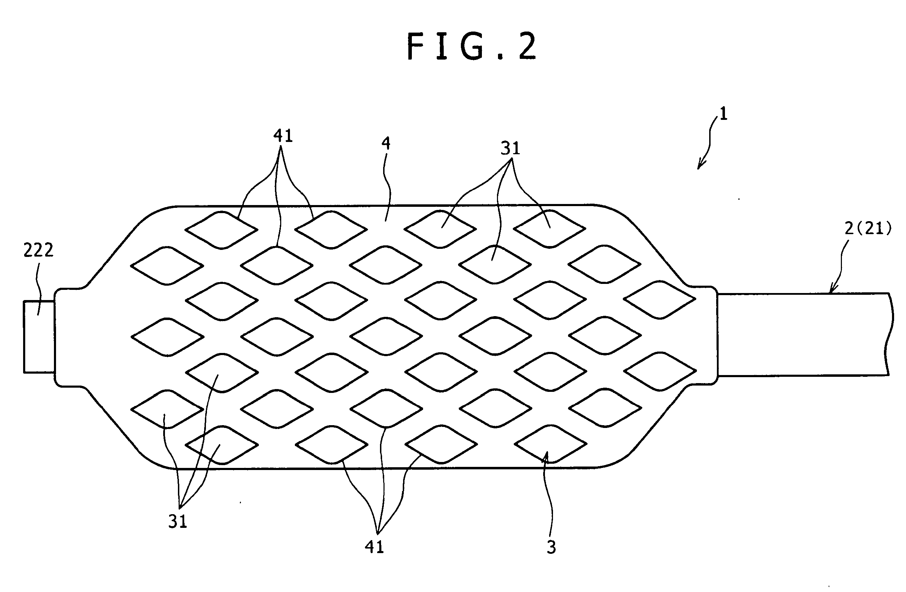

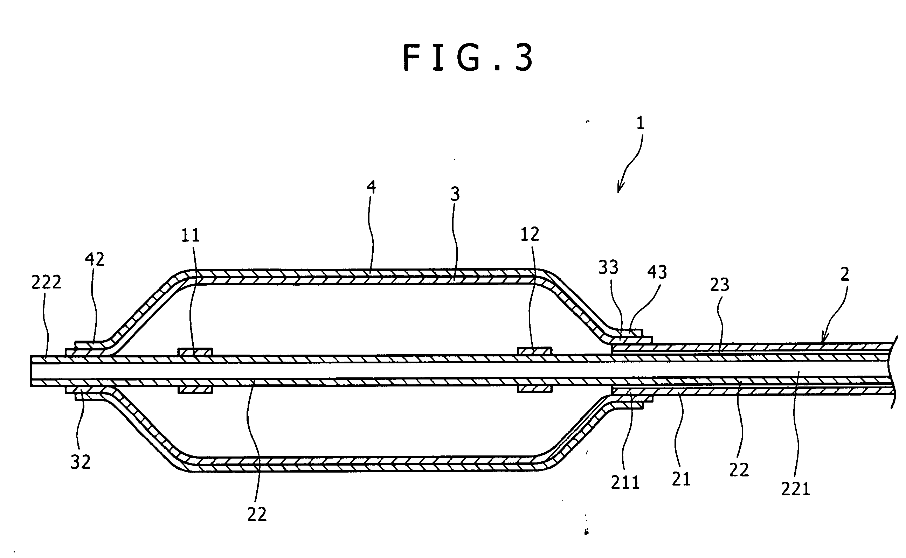

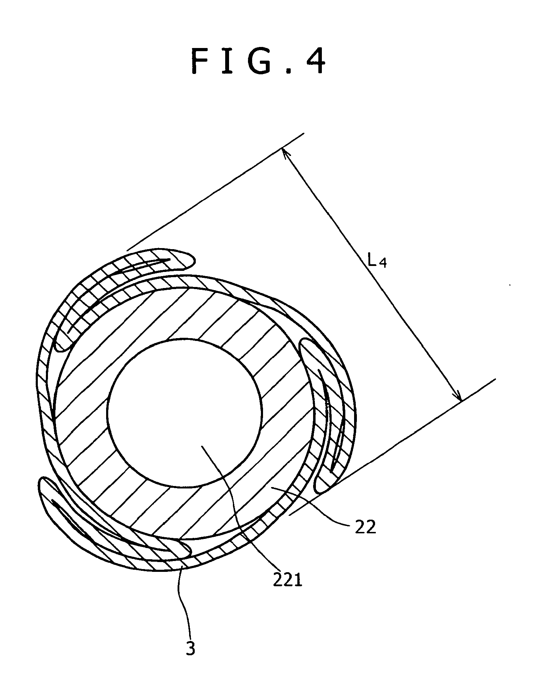

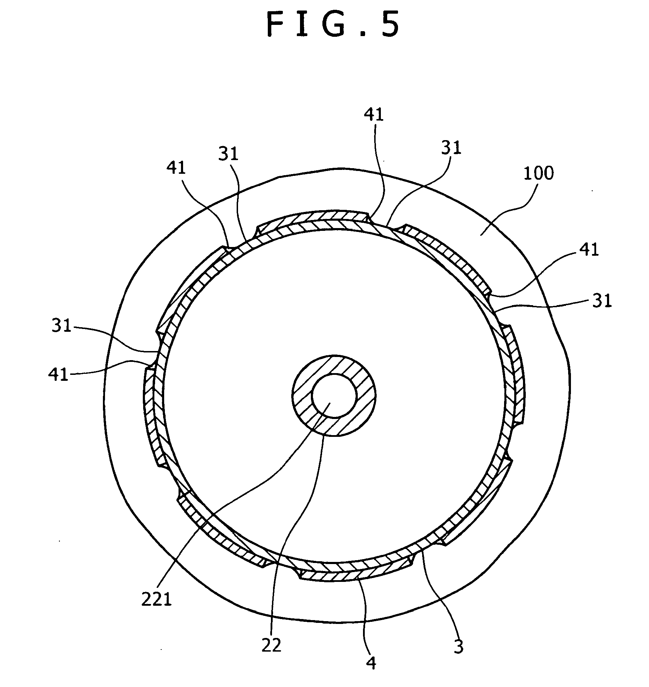

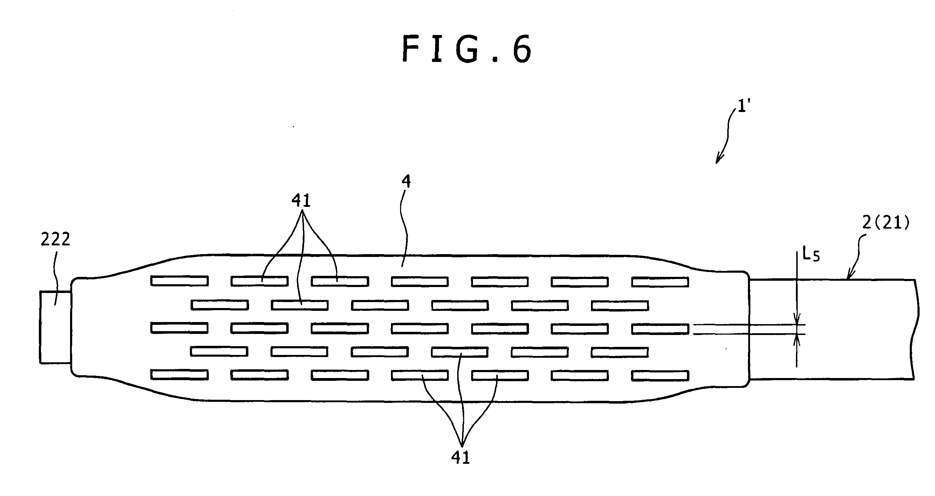

Fig. 1 is a side view showing the surroundings of an expandable body in its contracted state in a first embodiment of the catheter with the expandable body according to the present invention; Figs. 2 and 3 are respectively a side view and a vertical sectional view, showing the surroundings of the expandable body in its expanded state in the catheter with the expandable body shown in Fig. 1; Fig. 4 is a cross-sectional view of the expandable body in its contacted state and an inner tube, in the catheter with the expandable body shown in Fig. 1; Fig. 5 is a cross-sectional view of the surroundings of the expandable body in its expanded state in the catheter with the expandable body shown in Fig. 1; and Fig. 6 is a side view for illustrating another configuration example of a slit(s) in an outer sleeve. Incidentally, for convenience in description, the right side in Figs. 1 to 3 will hereinafter be referred to as "the proximal end", and the left side will be referred to as "the distal end".

The catheter with the expandable body, 1, shown in these figures is for use in a treatment including the step of dilating a constricted (stenosed) portion (lesion portion) 100 generated in a body lumen such as a blood vessel, and includes an elongate (slender) shaft (catheter main body) 2, an expandable body (balloon) 3 being capable of expansion and contraction and disposed at a distal end portion of the shaft 2, and a tubular outer sleeve 4 put on the outside of the expandable body 3. The whole length of the catheter with the expandable body, 1, is not particularly limited; in the case of a catheter with an expandable body for use in PTCA, ordinarily, the whole length is preferably in the range of 1200 to 1600 mm, more preferably 1300 to 1500 mm.

As shown in Fig. 3, the shaft 2 includes a tubular outer tube 21 having flexibility (elasticity), and a tubular inner tube 22 having flexibility (elasticity) and inserted,in a hollow portion (lumen) of the outer tube 21.

The material constituting the outer tube 21 is not particularly limited, and examples of the material include flexible high polymeric materials such as polyamide-based resins such as nylon 11, nylon 12, nylon 610, etc., polyamide elastomers, polyolefins such as polypropylene, polyethylene, etc., olefin-based elastomers such as polyethylene elastomer, polypropylene elastomer, etc., polyesters such as polyethylene terephthalate, etc., polyester elastomers, soft polyvinyl chloride, polyurethane and polyurethane elastomers, fluoro-resins and fluoro-resin-based elastomers such as polytetrafluoroethylene, etc., polyimides, ethylene-vinyl acetate copolymer, and silicone rubbers, and metallic materials such as stainless steels, titanium, titanium alloys, superelastic alloys such as TiNi alloy, etc., which may be used either singly or in combination of two or more of them.

The outside diameter of the outer tube 21 is not particularly limited. In the case of a catheter with an expandable body for use in PTCA, ordinarily, the outside diameter is preferably in the range of 0.70 to 1.1 mm, more preferably 0.80 to 0.90 mm.

The hollow portion of the inner tube 22 functions as a guide wire lumen 221 for permitting a guide wire (not shown) to pass therethrough.

The material constituting the inner tube 22 is not particularly limited, and preferred examples of the material include metallic materials such as stainless steels, titanium, titanium alloys, superelastic alloys such as TiNi alloy, etc., and high molecular materials such as polyamides such as nylon 12, etc., polyamide elastomers, fluoro-resins such as PTFE (polytetrafluoroethylene), ETFE, FEP, PFA, etc., polyethylene, and polyester-based resins.

The outside diameter of the inner tube 22 is not particularly limited. In the case of a catheter with an expandable body for use in PTCA, ordinarily, the outside diameter is preferably in the range of 0.40 to 0.80 mm, more preferably 0.50 to 0.60 mm.

A distal end portion 222 of the inner tube 22 protrudes in the distal end direction beyond a distal end portion 211 of the outer pipe 21.

Between the outer tube 21 and the inner tube 22, a passage (inflation lumen) 23 is formed through which a working fluid (expanding fluid) for expansion and contraction of the expandable body 3 can flow. Namely, the outside diameter of the inner tube 22 is smaller than the inside diameter of the outer tube 21.

At a proximal end portion of the shaft 2 as above, a hub (not shown) is disposed. The hub is provided with a port communicated with the guide wire lumen 221, and a port communicated with the passage 23. A balloon inflator (not shown), for example, a syringe, can be connected to the port communicated with the passage 23. The expandable body 3 can be expanded and contracted by feeding the working fluid, which is supplied from the balloon inflator, into the expandable body 3 through the passage 23 or draining the working fluid out. The working fluid is preferably a liquid. Among usable liquids, more preferred is a liquid having an X-ray contrast property, for example, a liquid obtained by diluting an X-ray contrast agent, such as a contrast agent for artery, with physiological saline in a factor of several folds.

The expandable body 3 is composed of a tubular film member having flexibility. A proximal end portion 33 of the expandable body 3 is attached liquid-tight to the vicinity of the distal end portion 211 of the outer tube 21 over the entire circumference, and a distal end portion 32 of the expandable body 3 is attached liquid-tight to the vicinity of the distal end portion 222 of the inner tube 22 over the entire circumference. Incidentally, the method for the attachment is not particularly limited, and examples of the method include fusing, adhesion by use of an adhesive, etc.

The material constituting the expandable body 3 is preferably biaxially stretchable plastics. Examples of the material constituting the expandable body 3 include polyamide-based resins such as nylon 11, nylon 12, nylon 610, etc., polyamide elasomers, , polyesters such as polyethylene terephthalate (PET), etc., polyester elastomers, natural rubber, polyethylene, polypropylene, ethylene-propylene copolymer, ethylene-vinyl acetate copolymer, soft polyvinyl chloride, polyurethane, polyisoprene, polyimides, polytetrafluoroethylene, silicones, polyether-ether ketone (PEEK), and polymer blends and polymer alloys containing at least one of these polymers.

The wall strength of the expandable body 3 is preferably in excess of 15000psi.

As shown in Fig. 4, the expandable body 3, in its contracted state (before expanded), is in the state of being folded on (being wound around) the periphery of the inner tube 22. Incidentally, in Fig. 4, the outer sleeve 4 is omitted.

With a working fluid fed into the expandable body 3 starting from this condition, the expandable body 3 is brought into the expanded state, as shown in Figs. 3 and 5.

The size of the expandable body 3 is not particularly limited. In the case of a catheter with an expandable body for use in PTCA, ordinarily, the diameter upon expansion is preferably in the range of φ1.0 to φ5.0 mm, more preferably φ 1.25 to φ 4.0 mm. The axial length of the expandable body 3 is preferably in the range of 5 to 100 mm, more preferably 10 to 50 mm.

As shown in Fig. 3, at an outer peripheral portion of the inner tube 22 located inside the expandable body 3, radiopaque markers 11, 12 having a contrast property (particularly in X-ray imaging) are disposed. The radiopaque markers 11, 12 are provided at positions indicating the boundaries between a hollow cylindrical portion and conical portions of the expandable body 3 in its expanded state. The radiopaque markers 11, 12 may each be composed, for example, of a thin wire, coil, or ring of gold, silver, platinum, or tungsten. With such radiopaque markers 11, 12 provided, the catheter can be inserted into a living body while confirming the position of the expandable body 3 under fluororoentgenography. The markers 11, 12 may be those which display a contrast property in other imaging methods than fluororoentgenography, for example, CT scan, MRI or the like, thereby permitting the confirmation of their positions.

On the outside of the expandable body 3, the outer sleeve 4 composed of a tubular film member is disposed so as to cover the expandable body 3.

As shown in Fig. 1, the outer sleeve 4 is provided with a plurality of slits 41. In this embodiment, the slits 41 are formed substantially in parallel to the longitudinal direction (axial direction) of the outer sleeve 4.

The plurality of slits 41 are formed (arranged) intermittently (at intervals) along the circumferential direction of the outer sleeve 4, as indicated by symbols 41a, 41b and 41c in Fig. 1.

Besides, the plurality of slits 41 are formed (arranged) intermittently (at intervals) along the longitudinal direction of the outer sleeve 4, as indicated by symbols 41d, 41e and 41f in Fig. 1.

The outer sleeve 4 as above has such a degree of extension/contraction (stretch) property as to permit expansion of the expandable body 3, and is expanded and contracted attendant on the expansion and contraction of the expandable body 3.

As shown in Fig. 2, the slits 41 are opened when the expandable body 3 is expanded. As a result, the outside surface 31 of the expandable body 3 is exposed via the openings of the opened slits 41.

Then, when the expandable body 3 is contracted, the slits 41 are again closed as shown in Fig. 1 due to the elasticity of the outer sleeve 4.

In the catheter with the expandable body, 1, according to the present invention, the provision of the outer sleeve 4 ensures that when the expandable body 3 once expanded is contracted by drawing the working fluid from the inside thereof, a compressive force is exerted on the expandable body 3 over the entire circumference thereof by the elasticity of the outer sleeve 4 and, therefore, the expandable body 3 can be contracted into a compact form. In the case of this embodiment, the expandable body 3 can be contracted so as to return assuredly to the folded state shown in Fig. 4.

Therefore, when the expandable body 3 is contracted from the expanded state, the expandable body 3 can be securely prevented from being squashed flat into a war fan-like (wing-like) shape without being folded. Accordingly, excellent operability (called re-insertability or recross property) can be obtained at the time when the catheter with the expandable body, 1, once evulsed from the constricted portion 100 is again inserted into the constricted portion 100, so that the re-insertion can be performed smoothly and easily.

Besides, in the present invention, the outer sleeve 4 is provided with the slits 41 which are opened upon expansion of the expandable body 3, so that the outer sleeve 4 can be easily bulged in the radial direction and, therefore, the expandable body 3 can be sufficiently expanded without being obstructed by the outer sleeve 4.

In addition, as shown in Fig. 5, at the time of expanding the expandable body 3 to thereby dilate the constricted portion 100 of a blood vessel, the contact surface, for contact with the constricted portion 100, formed by the outside surface 31 of the expandable body 3 exposed via the opened slits 41 and the outer sleeve 4 is rugged (is including projections and recesses), the friction between the contact surface and the constricted portion 100 is enhanced, and an anti-slipping effect is displayed. Therefore, the expandable body 3 can be securely prevented from slipping off from the constricted portion 100, even where the expandable body 3 is expanded with a high pressure.

The inside surface of the outer sleeve 4 is preferably in close contact with the outside surface 31 of the expandable body 3 even when the expandable body 3 is in its folded state (contracted state). In this case, the expandable body 3 can be contracted into the folded form more assuredly.

Further, the outer sleeve 4 is preferably frictionally fastened to the outside surface 31 of the expandable body 3 even when the expandable body 3 is in its folded state (contracted state). In this case, by a fastening force of the outer sleeve 4, the expandable body 3 can be contracted into the folded form more assuredly.

Particularly, where the inside diameter of the outer sleeve 4 in the natural state (in the condition where no external force is exerted on the outer sleeve 4 standing alone) is not more than the maximum outside diameter (the size denoted by L₄ in Fig. 4) of the expandable body 3 in its folded state, the fastening force of the outer sleeve 4 acts on the expandable body 3 assuredly, so that the expandable body 3 can be contracted into the folded shape particularly assuredly, which is desirable. Incidentally, the maximum outside diameter means the maximum one of the distances between arbitrary two portions of the expandable body 3 which are located on the opposite sides with the center axis of the shaft 2 (the inner tube 22) therebetween, in the cross section of the catheter with the expandable body, 1. Incidentally, in the case where the expandable body 3 has the same outside diameter over the entire circumference thereof in its folded (contracted) state, it is preferable that the inside diameter of the outer sleeve 4 in the natural state is not more than the outside diameter of the expandable body 3.

The fixation locations of the outer sleeve 4 are not particularly limited. In this embodiment, as shown in Fig. 3, a distal end portion 42 of the outer sleeve 4 is attached to a distal end portion 32 of the expandable body 3, and a proximal end portion 43 of the outer sleeve 4 is attached to a proximal end portion 33 of the expandable body 3. This makes it possible to prevent assuredly the outer sleeve 4 from slipping off from the expandable body 3, at the time of insertion into a body lumen such as a blood vessel. Incidentally, the method for fixation at the attached portions is not particularly limited, and may be, for example, fusing, adhesion by use of an adhesive, or the like.

In addition, the slits 41 are each preferably formed in parallel to the longitudinal direction of the outer sleeve 4 or at an inclination relative to the longitudinal direction. More preferably, as in this embodiment, the slits 41 are substantially in parallel to the longitudinal direction of the outer sleeve 4. This configuration makes it possible for the slits 41 to be opened more easily upon expansion of the expandable body 3.

Besides, in the present invention, the number of the slit(s) 41 may be one. In this embodiment, a plurality of the slits 41 are formed, and the slits 41 are arranged dispersely along the circumferential direction of the outer sleeve 4, whereby the expansion and contraction of the outer sleeve 4 are made smoother.

The material constituting the outer sleeve 4 is not particularly limited, and may be a material having a certain degree of flexibility. Preferable examples of the material include silicone rubbers, nylons (polyamides), nylon elastomers (polyamide elastomers), latex rubbers, and polyester elastomers. Besides, a material having a contrast property, such as barium sulfate, may be kneaded into the material constituting the outer sleeve 4.

At the time of producing the catheter with the expandable body, 1, the inside diameter of the outer sleeve 4 is enlarged by opening the slits 41 and, in this condition, the outer sleeve 4 can be easily put on the outside of the expandable body 3. Therefore, the catheter with the expandable body, 1, can be produced easily.

The method for forming the slits 41 is not particularly limited. The formation can be carried out, for example, by laser processing (e.g., excimer laser, YAG laser), discharge processing, chemical etching, cutting, or the like. Among these forming methods, preferred is the laser processing, from the viewpoints of easiness of formation of the slits 41 or small holes, excellent shape accuracy, and excellent dimensional accuracy. Among laser processings, particularly preferred is the processing by a laser of which the oscillation wavelength is in the UV region. Particularly, excimer laser is preferable. However, where the base material of the outer sleeve 4 is an elastic member and thermal deformation thereof by laser is expected, cutting by use of a sharp cutting tool is also preferred.

The dimensions of the outer sleeve 4 can be appropriately set according to the dimensions of the expandable body 3. For example, the length of the outer sleeve 4 is preferably at such a value as to cover at least the folded portion (the conical portions (balloon taper portions) in Fig. 3 and the hollow cylindrical portion (straight portion)) of the expandable body 3. Specifically, in the case of a catheter with an expandable body, 1, for use in PTCA for dilating a constricted portion of the coronary artery, the length of the outer sleeve 4 is preferably in the range of about 1 to 100 mm, more preferably about 10 to 50 mm. In addition, the material thickness of the outer sleeve 4 in the natural state is typically in the range of about 1 to 1000 µm, preferably about 5 to 100µm, more preferably about 5 to 20 *µ*m. The upper limit of the thickness of the sleeve 4 is set from the standpoint of ensuring contact of both the outer sleeve 4 and the outside surface 31 of the expandable body 3 with the constricted portion 100 so as to dilate the constricted portion steadily. On the contrary, the lower limit of the thickness of the sleeve 4 is set from the standpoint of improving the anti-slipping effect with sufficient vertical interval between the outside surface of the sleeve 4 and the outside surface 31 of.the expandable body 3 exposed via the opened slits 41 so as to enhance the friction between the contact surface formed by the outer sleeve 4 and the outside surface 31 of the expandable body 3 and the constricted portion 100 sufficiently.Besides, the inside diameter of the outer sleeve 4 in the natural state is preferably in the range of about 0.4 to 2.0 mm, more preferably about 0.5 to 1.0 mm.

In this embodiment, the length L₁ (see Fig. 1) of each slit 41 is preferably in the range of about 0.5 to 5 mm, more preferably about 1 to 4 mm.

In addition, the pitch L₂ (see Fig. 1) of the slits 41 along the circumferential direction of the outer sleeve 4 is preferably in the range of about 0.1 to 5 mm, more preferably about 0.5 to 2 mm.

Besides, the pitch L₃ (see Fig. 1) of the slits 41 along the longitudinal direction of the outer sleeve 4 is preferably in the range of about 0.6 to 9.9 mm, more preferably about 1.5 to 6 mm.

The slits 41 may be composed of crevices which close substantially completely upon contraction of the expandable body 3, as shown in Fig. 1, or may not close completely but have a width L₅ upon contraction of the expandable body 3, like slits 41 in a catheter with an expandable body, 1', shown in Fig. 6. In the case as shown in Fig. 6, the width L₅ of the slits 41 upon contraction of the expandable body 3 is preferably not more than 2 mm, more preferably not more than 1 mm.

As in the configuration shown in Fig. 1, the slits 41 are preferably so arranged that the slits 41 adjacent to each other in the circumferential direction of the expandable body 3 are deviated from each other in the longitudinal direction of the outer sleeve 4. This ensures that, at the time of expansion of the expandable body 3, the outer sleeve 4 is more easily expanded at any portion over the entire part in the longitudinal direction thereof, so that the outer sleeve 4 can be expanded uniformly, as shown in Fig. 2. Further, the slits 41 are preferably so arranged that at least one slit 41 is present at any portion in the longitudinal direction of the outer sleeve 4. This ensures that, at the time of expansion of the expandable body 3, the outer sleeve 4 can be expanded more assuredly over the entire part thereof.

In the catheter with the expandable body, 1, it is preferable that at least one of the outside surface of the outer sleeve 4 and the outside surface 31 of the expandable body 3 is subjected to a high lubricity treatment for displaying a comparatively high lubricity, and the other is subjected to a low lubricity treatment for displaying a comparatively lower lubricity or is not subjected to any lubricity treatment.

This ensures that, when the expandable body 3 is expanded, a poor lubricity surface is present in the contact surface, for contact with the constricted portion 100, formed by the expandable body 3 and the outer sleeve 4, with the result that the friction between the contact surface and the constricted portion 100 can be enhanced. Therefore, even where the expandable body 3 is expanded with a high pressure, it is possible to prevent more securely the expandable body 3 from slipping off from the constricted portion 100.

In this case, it is preferable that the outside surface of the outer sleeve 4 is subjected to the high lubricity treatment, and the outside surface 31 of the expandable body 3 is subjected to the low lubricity treatment. It is more preferable that the outside surface of the outer sleeve 4 is subjected to the high lubricity treatment, and the outside surface 31 of the expandable body 3 is not subjected to any lubricity treatment. This results in that, when the expandable body 3 is contracted for insertion into a body lumen such as a blood vessel or for evulsion from the body lumen, the outside surface 31 of the expandable body 3 which is poor in lubricity is covered with the outer sleeve 4, and only the outside surface of the outer sleeve 4 which is high in lubricity is exposed to the outside of the catheter with the expandable body, 1, and makes contact with the inside wall of the body lumen. Thus, it is possible to provide a catheter with an expandable body, 1, which is excellent in the property for insertion into a body lumen and in the property for evulsion from the body lumen.

On the other hand, where the outside surface 31 of the expandable body 3 is subjected to the low lubricity treatment, the friction between the mutually contacting portions of the outside surface 31 of the folded expandable body 3 and the friction between the outside surface 31 of the inside surface of the sleeve 4 can be reduced. This ensures that, even if the sleeve 4 is making close contact with the expandable body 3 or is fastening the expandable body 3 at the time of expansion of the expandable body 3, the mutually contacting portions of the outside surface 31 of the expandable body 3 or the inside surface of the sleeve 4 and the outside surface 31 or the expandable body 3 will easily slip on each other, so that the expandable body 3 can be expanded more smoothly.

In addition, where the inside surface of the sleeve 4 is subjected to a lubricity treatment, the friction between the outside surface 31 of the expandable body 3 and the inside surface of the sleeve 4 can be reduced. This ensures that, even if the sleeve 4 is making close contact with the expandable body 3 or is fastening the expandable body 3, the outside surface 31 of the expandable body 3 will easily slip relative to the sleeve 4 at the time of expansion, so that the expandable body 3 can be expanded more smoothly. In this case, the outside surface 31 of the expandable body 3 may be subjected to a lubricity treatment, but it is preferable not to subject the outside surface 31 to any lubricity treatment, since the friction between the outside surface 31 and the constricted portion 100 is enhanced and slipping therebetween is prevented, at the time of expansion.

Examples of the high lubricity treatment include application (formation of a coating layer) of a hydrophilic high polymeric material showing lubricity upon wetting (absorption of water). Examples of the hydrophilic high polymeric material include cellulose-based high polymeric materials, polyethylene oxide-based high polymeric materials, maleic anhydride-based high polymeric materials (e.g., maleic anhydride copolymer such as methyl vinyl ether-maleic anhydride copolymer), acrylamide-based high polymeric materials (e.g., polyacrylamide, polyglycidyl methacrylate-dimethylacrylamide (PGMA-DMAA) block copolymer), watersoluble nylons, polyvinyl alcohol, and polyvinyl pyrrolidone.

Besides, examples of the low lubricity treatment include silicone coating, PTFE coating, and the like methods.

Incidentally, such a lubricity-imparting treatment as above may be applied also to the outer peripheral surface of the outer tube 21. This ensures that, at the time of inserting the catheter with the expandable body, 1, into a blood vessel 100, the friction is lowered, the insertion can be performed more smoothly, and operability and safety are enhanced.

### <Second Embodiment>

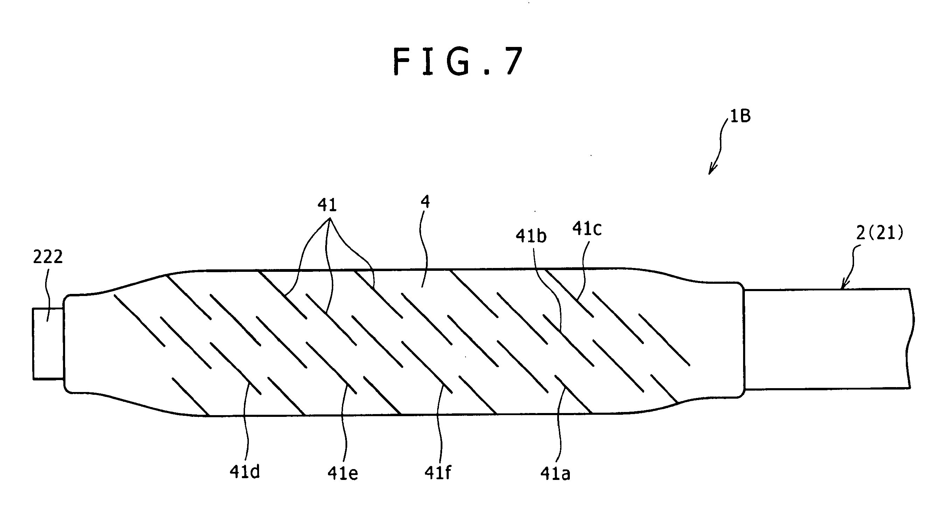

Fig. 7 is a side view showing the surroundings of an expandable body in its contracted state in a second embodiment of the catheter with the expandable body according to the present invention.

Now, the second embodiment of the catheter with the expandable body according to the present invention will be described below referring to this figure. The following description will be centered on differences from the first embodiment above, and description of the same points as in the first embodiment will be omitted.

The catheter with the expandable body, 1B, according to this embodiment is the same as the first embodiment, except that slits 41 are formed at an inclination relative to the longitudinal direction of the outer sleeve 4.

A plurality of the slits 41 are formed (arranged) intermittently (at intervals) along the circumferential direction of the outer sleeve 4 as indicated by symbols 41a, 41b and 41c in Fig. 7, and are formed (arranged) intermittently (at intervals) also along the longitudinal direction of the outer sleeve 4 as indicated by symbols 41d, 41e and 41f in Fig. 7.

In this embodiment, the same effects as in the first embodiment above can be obtained.

### <Third Embodiment>

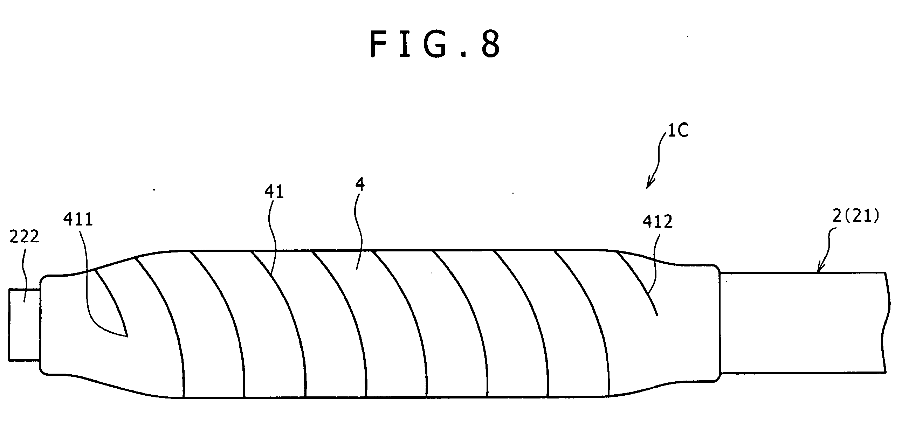

Fig. 8 is a side view showing the surroundings of an expandable body in its contracted state in a third embodiment of the catheter with the expandable body according to the present invention.

Now, the third embodiment of the catheter with the expandable body according to the present invention will be described below referring to this figure. The following description will be centered on differences from the first embodiment above, and description of the same points as in the first embodiment will be omitted.

In the catheter with the expandable body, 1C, according to this embodiment, one slit 41 is formed in a spiral shape with the center axis of the outer sleeve 4 as a center. This ensures that, in this embodiment, stress concentration would not occur in the vicinity of end portions of the slit 41 in the outer sleeve 4 upon expansion of the expandable body 3. Therefore, even where the outer sleeve 4 is formed of a material having a comparatively low strength or where the expandable body 3 is expanded with a high pressure, it is possible to securely prevent cracks from being generated in the vicinity of the end portions of the slit 41 at the time of expansion of the expandable body 3.

In addition, the slit 41 is absent in the vicinity of the distal end of the outer sleeve 4 and in the vicinity of the proximal end of the outer sleeve 4. In other words, the tipmost end 411 of the slit 41 is spaced to the proximal end side from the distal end of the outer sleeve 4, and the basemost end 412 of the slit 41 is spaced to the distal end side from the proximal end of the outer sleeve 4. This ensures that the fixation to the outer tube 21 and the inner tube 22 can be performed more uniformly.

Incidentally, in this embodiment, a plurality of spiral slits 41 may be formed in the shape of a multiple spiral.

### <Fourth Embodiment>

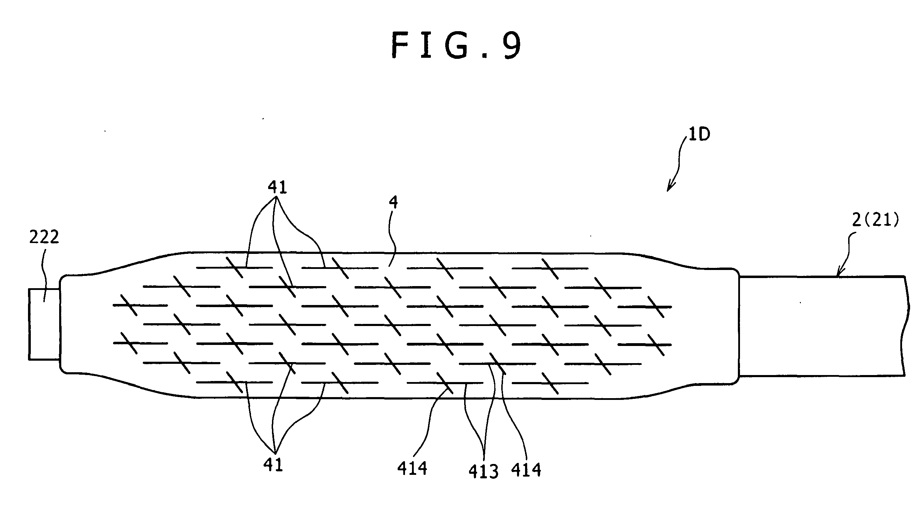

Fig. 9 is a side view showing the surroundings of an expandable body in its contracted state in a fourth embodiment of the catheter with the expandable body according to the present invention.

Now, the fourth embodiment of the catheter with the expandable body according to the present invention will be described below referring to this figure. The following description will be centered on differences from the first embodiment above, and description of the same points as in the first embodiment will be omitted.

The catheter with the expandable body, 1D, according to this embodiment is the same as the first embodiment, except that the shape of slits 41 is different.

In this embodiment, each of the slits 41 has a shape in which a parallel portion 413 parallel to the longitudinal direction of the outer sleeve 4 and an inclined portion 414 inclined relative to the longitudinal direction intersect each other. This makes it possible for the slits 41 to be opened more largely and easily at the time of expansion of the expandable body 3.

### <Fifth Embodiment>

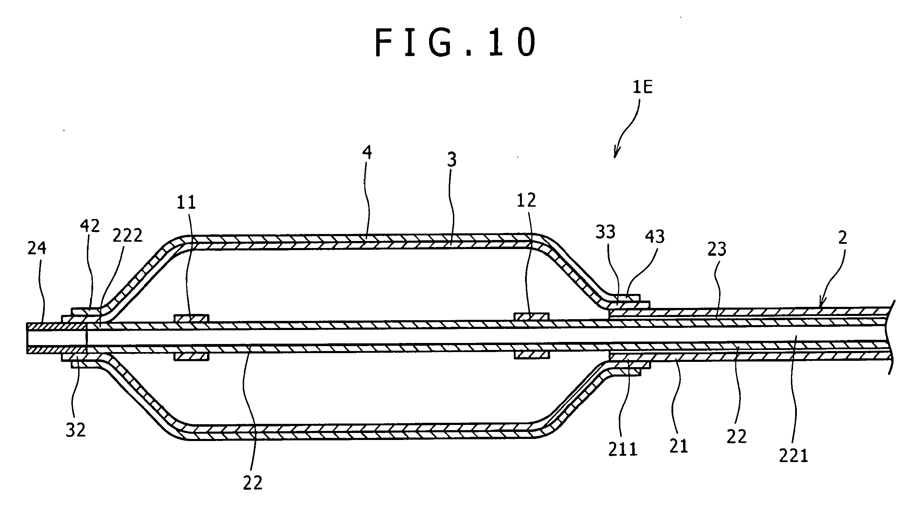

Fig. 10 is a vertical sectional view showing the surroundings of an expandable body in its expanded state in a fifth embodiment of the catheter with the expandable body according to the present invention.

Now, the fifth embodiment of the catheter with the expandable body according to the present invention will be described below referring to this figure. The following description will be centered on differences from the first embodiment above, and description of the same points as in the first embodiment will be omitted.

A shaft 2 of the catheter with the expandable body, 1E, according to this embodiment has a tubular distal end tip 24 joined to a distal end portion 222 of an inner tube 22. The distal end tip 24 is higher in flexibility than the inner tube 22, and its inside and outside diameters are nearly equal to those of the inner tube 22, respectively.

A distal end portion 32 of the expandable body 3 is attached liquid-tight to the distal end portion 222 of the inner tube 22 and the distal end tip 24 over the entire circumference.

In this embodiment, the provision of the flexible distal end tip 24 makes it possible to further alleviate the stimulus to the inside wall of a body lumen at the time of inserting the catheter with the expandable body, 1E, into the body lumen, so that a higher safety can be obtained.

### <Sixth Embodiment>

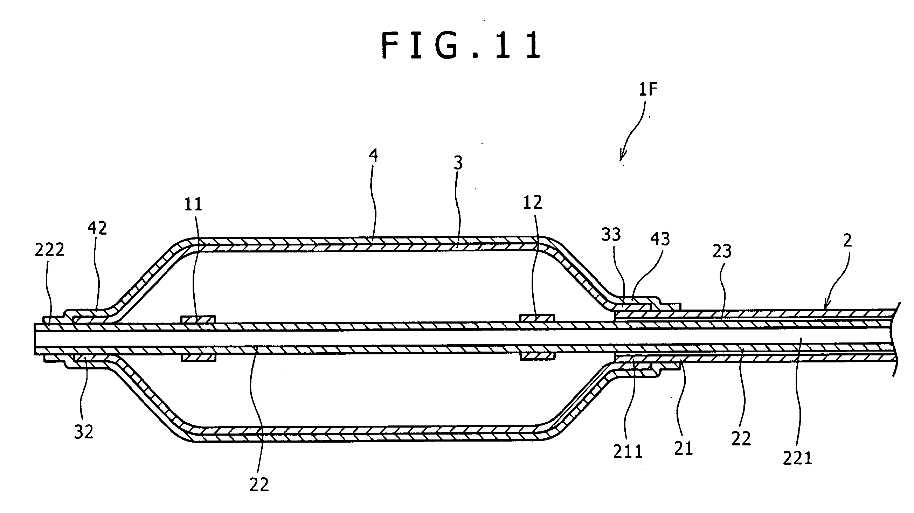

Fig. 11 is a vertical sectional view showing the surroundings of an expandable body in its expanded state in a sixth embodiment of the catheter with the expandable body according to the present invention.

Now, the sixth embodiment of the catheter with the expandable body according to the present invention will be described below referring to this figure. The following description will be centered on differences from the first embodiment above, and description of the same points as in the first embodiment will be omitted.

In the catheter with the expandable body, 1F, according to the present invention, a distal end portion 42 of the outer sleeve 4 is extended to the distal end side beyond a distal end portion 32 of the expandable body 3. The distal end portion 42 of the outer sleeve 4 is attached to both the distal end portion 32 of the expandable body 3 and the vicinity of a distal end portion 222 of the inner tube 22. In addition, a proximal end portion 43 of the outer sleeve 4 is extended to the proximal end side beyond a proximal end portion 33 of the expandable body 3. The proximal end portion 43 of the outer sleeve is attached to both the proximal end portion 33 of the expandable body 3 and a distal end portion 211 of the outer tube 21.

This configuration ensures that, in the catheter with the expandable body, 1F, the outer sleeve 4 can be fixed more firmly, and exfoliation of fixed portions and the like troubles can be prevented more securely.

### <Seventh Embodiment>

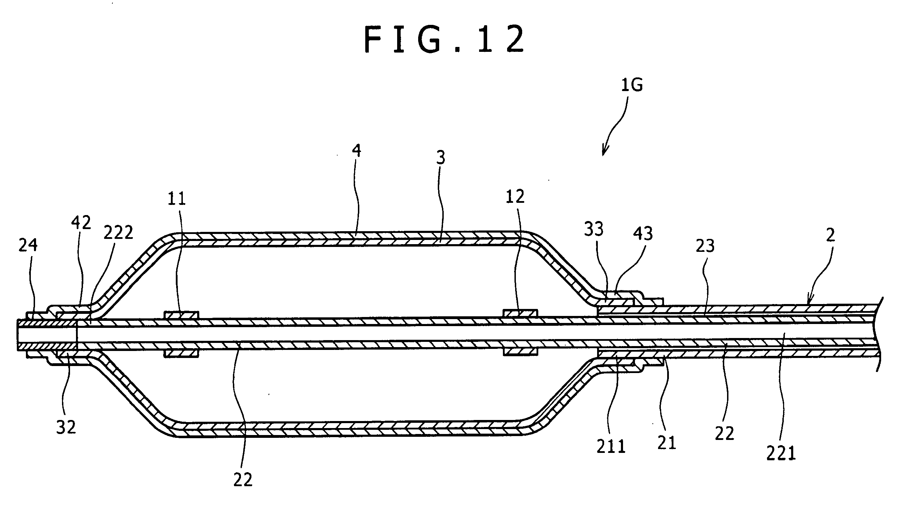

Fig. 12 is a vertical sectional view showing the surroundings of an expandable body in its expanded state in a seventh embodiment of the catheter with the expandable body according to the present invention.

Now, the seventh embodiment of the catheter with the expandable body according to the present invention will be described below referring to this figure. The following description will be centered on differences from the first embodiment above, and description of the same points as in the first embodiment will be omitted.

A shaft 2 in the catheter with the expandable body, 1G, according to this embodiment has a tubular distal end tip 24 joined to a distal end portion 222 of the inner tube 22. The distal end tip 24 is higher in flexibility than the inner tube 22, and its inside and outside diameters are nearly equal to those of the inner tube 22, respectively.

A distal end portion 32 of the expandable body 3 is attached liquid-tight to a distal end portion 222 of the inner tube 22 and the distal end tip 24 over the entire circumference.

In this embodiment, the provision of the flexible distal end tip 24 makes it possible to further alleviate the stimulus to the inside wall of a body lumen at the time of inserting the catheter with the expandable body, 1G, into the body lumen, so that a higher safety can be obtained.

In addition, in the catheter with the expandable body, 1G, according to this embodiment, a distal end portion 42 of the outer sleeve 4 is extended to the distal end side beyond the distal end portion of the expandable body 3. The distal end portion 42 of the outer sleeve 4 is attached to both the distal end portion 32 of the expandable body 3 and the distal end tip 24. Besides, a proximal end portion 43 of the outer sleeve 4 is extended to the proximal end side beyond a proximal end portion 33 of the expandable body 3. The proximal end portion 43 of the outer sleeve 4 is attached to both the proximal end portion 33 of the expandable body 3 and a distal end portion 211 of the outer tube 21.

This configuration ensures that, in the catheter with the expandable body, 1G, the outer sleeve 4 can be fixed more firmly, and exfoliation of fixed portions or the like troubles can be prevented more assuredly.

While the embodiments of the catheter with the expandable body according to the present invention have been described above, arbitrary two or more configurations (characteristics) of these embodiments may be combined with each other in the present invention.

In addition, a difference or differences in the manner of formation of the slit(s) 41 may be provided between a distal end side portion and a proximal end side portion of the outer sleeve 4. Here, the difference or differences in the manner of formation of the slit(s) 41 mean a difference or differences in the length (L₁) of the slit(s) 41, the width (L₅) of the slit(s) 41, the direction (inclination angle) of the slit(s) 41, the shape of the slit(s) 41, the formation density of the slit(s) 41, and the like.

For example, in the case of the configuration as in Fig. 1, when the formation density of the slits 41 is set higher (the pitch L₂ set smaller) in a distal end side portion (in the longitudinal direction) of the outer sleeve 4 and the formation density of the slits 41 is set lower (the pitch L₂ set larger) in a proximal end side portion (in the longitudinal direction) of the outer sleeve 4, the flexibility of the distal end side portion of the sleeve 4 is enhanced, so that the sleeve 4 (and the expandable body 3) will be easily expanded at the distal end side portion, and the property for passing to a constricted portion 100 is enhanced.

Other than the above example, a difference or differences in the manner of formation of the slit(s) 41 may be provided between the vicinity of both ends and a central portion in the longitudinal direction of the outer sleeve 4, whereby a diversity of functions can be added in accordance with the individual cases.

While the catheter with the expandable body according to the present invention has been described above by way of the embodiments shown in the figures, the invention is not limited to the above embodiments, and each of the portions constituting the catheter with the expandable body can be replaced by one having an arbitrary configuration which can display the same function as the above-described. Besides, an arbitrary component or components may be added.

## Claims

1. A catheter comprising :
a shaft having a passage (23) for permitting a working fluid to pass therethrough,
an expandable body (3) which is capable of expansion and contraction and provided at a distal end portion of said shaft so as to be communicated with the inside of said passage, **characterized in that**
said catheter further comprises an elastic tubular outer sleeve (4) provided with at least one slit (41) and fastened to the outside of said expandable body, said tubular outer sleeve (4) having such a degree of extension/contraction property as to permit expansion of said expandable body when said working fluid is made to flow through said passage into said expandable body and to permit elastic contraction when said working fluid is sucked out of the expandable body to contract said expandable body (3) into a compact form.

2. A catheter with an expandable body according to claim 1, wherein said outer sleeve (4) is in close contact with the outside surface of said expandable body.

3. A catheter with an expandable body according to claim 1 or 2, wherein said expandable body (3) is provided in the state of being folded on the distal end portion of said shaft before expanded.

4. A catheter with an expandable body according to claim 3, wherein the inside diameter of said outer sleeve (4) in the natural state is not more than the maximum outside diameter of said expandable body in the folded state.

5. A catheter with an expandable body according to any of claims 1 to 4, wherein a distal end portion of said outer sleeve (4) is attached to a distal end portion of said expandable body and/or said shaft.

6. A catheter with an expandable body according to claims 1 to 5, wherein a proximal end portion of said outer sleeve (4) is attached to a proximal end portion of said expandable body and/or said shaft.

7. A catheter with an expandable body according to any of claims 1 to 6, wherein said slit is formed in parallel to the longitudinal direction of said outer sleeve or at an inclination relative to said longitudinal direction.

8. A catheter with an expandable body according to claim 7, wherein said outer sleeve (4) is provided with a plurality of said slits arranged intermittently along the circumferential direction of said outer sleeve.

9. A catheter with an expandable body according to claim 7 or 8, wherein said outer sleeve (4) is provided with a plurality of said slits arranged intermittently along the longitudinal direction of said outer sleeve.

10. A catheter with an expandable body according to any of claims 1 to 6, wherein said slit is formed in a spiral form, with the center axis of said outer sleeve as a center.

11. A catheter with an expandable body according to any of claims 1 to 10, wherein one of the outside surface of said outer sleeve and the outside surface of said expandable body has been subjected to a high lubricity treatment for displaying a comparatively high lubricity, and the other has been subjected to a low lubricity treatment for displaying a comparatively lower lubricity or has not been subjected to any lubricity treatment.

12. A catheter with an expandable body according to claim 11, wherein the outside surface of said outer sleeve has been subjected to said high lubricity treatment for displaying a comparatively high lubricity, and the outside surface of said expandable body has been subjected to said low lubricity treatment for displaying a comparatively lower lubricity.

13. A catheter with an expandable body according to claim 12, wherein the outside surface of said outer sleeve has been subjected to a lubricity treatment for displaying a lubricity, and the outside surface of said expandable body has not been subjected to any lubtricity treatment.

14. A catheter with an expandable body according to any of claims 1 to 14, wherein a difference in the manner of formation of said slits(s) exists between a distal end side portion and a proximal end side portion of said outer sleeve.

15. A catheter with an expandable body according to claim 14, wherein said difference in the manner of formation of said slits(s) is a difference or differences in at least one of the length, the width, the direction, the shape, and the formation density, of said slit(s).

## Patentansprüche

1. Katheter, umfassend:
einen Schaft, welcher einen Kanal (23) aufweist, um zu ermöglichen, daß ein Arbeitsfluid dadurch hindurch läuft,
einen ausdehnbaren Körper (3), welcher zur Expansion und Kontraktion geeignet ist und an einem distalen Endabschnitt des Schafts derart vorgesehen ist, daß dieser mit der Innenseite des Kanals verbunden ist, **dadurch gekennzeichnet, daß**
der Katheter einen elastischen rohrförmigen äußeren Mantel (4) umfaßt, welcher mit mindestens einem Schlitz (41) versehen und gegen die Außenseite des ausdehnbaren Körpers gespannt ist, wobei der rohrförmige äußere Mantel (4) ein geeignetes Maß einer Expansions-Kontraktions-Eigenschaft aufweist, um eine Ausdehnung des ausdehnbaren Körpers zu ermöglichen, wenn veranlaßt wird, daß das Arbeitsfluid durch den Kanal in den ausdehnbaren Körper fließt, und eine elastische Kontraktion zu ermöglichen, wenn das Arbeitsfluid aus dem ausdehnbaren Körper abgesaugt wird, um den ausdehnbaren Körper (3) zu einer kompakten Form zusammenzuziehen.

2. Katheter mit einem ausdehnbaren Körper nach Anspruch 1, wobei sich der äußere Mantel (4) in engem Kontakt mit der äußeren Oberfläche des ausdehnbaren Körpers befindet.

3. Katheter mit einem ausdehnbaren Körper nach Anspruch 1 oder 2, wobei der ausdehnbare Körper (3) in dem Zustand vorgesehen ist, daß dieser um den distalen Endabschnitts des Schafts gefaltet ist, bevor dieser ausgedehnt wird.

4. Katheter mit einem ausdehnbaren Körper nach Anspruch 3, wobei der Innendurchmesser des äußeren Mantels (4) in dem natürlichen Zustand nicht größer als der maximale Außendurchmesser des ausdehnbaren Körpers in dem gefalteten Zustand ist.

5. Katheter mit einem ausdehnbaren Körper nach einem der Ansprüche 1 bis 4, wobei ein distaler Endabschnitt des äußeren Mantels (4) an einem distalen Endabschnitt des ausdehnbaren Körpers und/oder des Schafts angebracht ist.

6. Katheter mit einem ausdehnbaren Körper nach Anspruch 1 bis 5, wobei ein proximaler Endabschnitt des äußeren Mantels (4) an einem proximalen Endabschnitt des ausdehnbaren Körpers und/oder des Schafts angebracht ist.

7. Katheter mit einem ausdehnbaren Körper nach einem der Ansprüche 1 bis 6, wobei der Schlitz parallel zu der Längsrichtung des äußeren Mantels oder mit einer Neigung bezüglich der Längsrichtung des äußeren Mantels bzw. mit einer Neigung bezüglich der Längsrichtung ausgebildet ist.

8. Katheter mit einem ausdehnbaren Körper nach Anspruch 7, wobei der äußere Mantel (4) mit einer Vielzahl der Schlitze versehen ist, welche mit Zwischenräumen entlang der Umfangsrichtung des äußeren Mantels angeordnet sind.

9. Katheter mit einem ausdehnbaren Körper nach Anspruch 7 oder 8, wobei der äußere Mantel (4) mit einer Vielzahl der Schlitze versehen ist, welche mit Zwischenräumen entlang der Längsrichtung des äußeren Mantels angeordnet sind.

10. Katheter mit einem ausdehnbaren Körper nach einem der Ansprüche 1 bis 6, wobei der Schlitz in einer Spiralenform mit der Mittelachse des äußeren Mantels als Mitte ausgebildet ist.

11. Katheter mit einem ausdehnbaren Körper nach einem der Ansprüche 1 bis 10, wobei eine Oberfläche aus der Gruppe der äußeren Oberfläche des äußeren Mantels und der äußeren Oberfläche des ausdehnbaren Körpers einer Behandlung zum Herstellen einer hohen Gleitfähigkeit zum Erzeugen einer relativ hohen Gleitfähigkeit unterzogen wurde und die andere einer Behandlung zum Herstellen einer niedrigen Gleitfähigkeit zum Erzeugen einer vergleichsweise niedrigeren Gleitfähigkeit unterzogen wurde oder keiner Gleitfähigkeitsbehandlung unterzogen wurde.

12. Katheter mit einem ausdehnbaren Körper nach Anspruch 11, wobei die äußere Oberfläche des äußeren Mantels der Behandlung zum Herstellen einer hohen Gleitfähigkeit zum Erzeugen einer relativ hohen Gleitfähigkeit unterzogen wurde und die äußere Oberfläche des ausdehnbaren Körpers der Behandlung zum Herstellen einer niedrigen Gleitfähigkeit zum Erzeugen einer vergleichsweise niedrigeren Gleitfähigkeit unterzogen wurde.

13. Katheter mit einem ausdehnbaren Körper nach Anspruch 12, wobei die äußere Oberfläche des äußeren Mantels einer Gleitfähigkeitsbehandlung zum Erzeugen Gleitfähigkeit unterzogen wurde und die äußere Oberfläche des ausdehnbaren Körpers keiner Gleitfähigkeitsbehandlung unterzogen wurde.

14. Katheter mit einem ausdehnbaren Körper nach einem der Ansprüche 1 bis 14, wobei ein Unterschied in der Ausbildungsweise des Schlitzes (der Schlitze) zwischen einem Abschnitt der Seite des distalen Endes und einem Abschnitt der Seite des proximalen Endes besteht.

15. Katheter mit einem ausdehnbaren Körper nach Anspruch 14, wobei der Unterschied in der Ausbildungsweise des Schlitzes (der Schlitze) einen Unterschied bzw. Unterschiede in mindestens einem Aspekt aus der Gruppe der Länge, der Breite, der Richtung der Gestalt und der Ausbildungsdichte des Schlitzes (der Schlitze) darstellt.

## Revendications

1. Cathéter comprenant :
un axe comportant un conduit (23) permettant le passage d'un fluide de travail,
un corps expansible (3) capable de s'expanser et de se contracter et placé dans une partie d'extrémité distale dudit axe pour pouvoir être mis en communication avec l'intérieur dudit conduit,
**caractérisé en ce que**
ledit cathéter comprend en outre un manchon extérieur tubulaire élastique (4) pourvu d'au moins une fente (41) et fixé sur l'extérieur dudit corps expansible, ledit manchon extérieur tubulaire (4) ayant un niveau de capacité d'extension/contraction prévu pour permettre l'expansion dudit corps expansible lorsque l'on fait circuler ledit fluide de travail dans ledit conduit et dans ledit corps expansible et pour permettre la contraction élastique lorsque ledit fluide de travail est aspiré hors du corps expansible pour contracter ledit corps expansible (3) sous une forme compacte.

2. Cathéter avec corps expansible selon la revendication 1, dans lequel ledit manchon extérieur (4) est en contact étroit avec la surface extérieure dudit corps expansible.

3. Cathéter avec corps expansible selon la revendication 1 ou 2, dans lequel ledit corps expansible (3) est fourni dans un état dans lequel il est plié sur la partie d'extrémité distale dudit axe avant d'être expansé.

4. Cathéter avec corps expansible selon la revendication 3, dans lequel le diamètre intérieur dudit manchon extérieur (4) dans l'état naturel n'est pas supérieur au diamètre extérieur maximum dudit corps expansible dans l'état plié.

5. Cathéter avec corps expansible selon l'une quelconque des revendications 1 à 4, dans lequel une partie d'extrémité distale dudit manchon extérieur (4) est attachée à une partie d'extrémité distale dudit corps expansible et/ou dudit axe.

6. Cathéter avec corps expansible selon les revendications 1 à 5, dans lequel une partie d'extrémité proximale dudit manchon extérieur (4) est attachée à une partie d'extrémité proximale dudit corps expansible et/ou dudit axe.

7. Cathéter avec corps expansible selon l'une quelconque des revendications 1 à 6, dans lequel ladite fente est formée parallèlement à la direction longitudinale dudit manchon extérieur ou avec une inclinaison par rapport à ladite direction longitudinale.

8. Cathéter avec corps expansible selon la revendication 7, dans lequel ledit manchon extérieur (4) est pourvu d'une pluralité desdites fentes disposées de façon intermittente le long de la direction circonférentielle dudit manchon extérieur.

9. Cathéter avec corps expansible selon la revendication 7 ou 8, dans lequel ledit manchon extérieur (4) est pourvu d'une pluralité desdites fentes disposées de façon intermittente le long de la direction longitudinale dudit manchon extérieur.

10. Cathéter avec corps expansible selon l'une quelconque des revendications 1 à 6, dans lequel ladite fente est en forme de spirale, ayant l'axe dudit manchon extérieur comme centre.

11. Cathéter avec corps expansible selon l'une quelconque des revendications 1 à 10, dans lequel une surface parmi la surface extérieure dudit manchon extérieur et la surface extérieure dudit corps expansible a été soumise à un traitement à haut pouvoir lubrifiant pour présenter un pouvoir lubrifiant relativement élevé, et l'autre a été soumise à un traitement à bas pouvoir lubrifiant pour présenter un pouvoir lubrifiant relativement bas ou bien n'a été soumise à aucun traitement lubrifiant.

12. Cathéter avec corps expansible selon la revendication 11, dans lequel la surface extérieure dudit manchon extérieur a été soumise audit traitement à haut pouvoir lubrifiant pour présenter un pouvoir lubrifiant relativement élevé, et la surface extérieure dudit corps expansible a été soumise audit traitement à bas pouvoir lubrifiant pour présenter un pouvoir lubrifiant relativement bas.

13. Cathéter avec corps expansible selon la revendication 12, dans lequel la surface extérieure dudit manchon extérieur a été soumise à un traitement lubrifiant pour présenter un pouvoir lubrifiant, et la surface extérieure dudit corps expansible n'a été soumise à aucun traitement lubrifiant.

14. Cathéter avec corps expansible selon l'une quelconque des revendications 1 à 13, dans lequel il existe une différence dans la manière de former la ou lesdites fente(s) entre une partie de côté d'extrémité distale et une partie de côté d'extrémité proximale dudit manchon extérieur.

15. Cathéter avec corps expansible selon la revendication 14, dans lequel ladite différence dans la manière de former la ou lesdites fente(s) est une ou des différence(s) dans au moins paramètre parmi la longueur, la largeur, la direction, la forme et la densité de formation de la ou lesdites fente(s).
